# EUROPEAN PATENT APPLICATION

(11) **EP 3 531 120 A1**
(43) Date of publication of application: **28.08.2019**
(21) Application number: 17865861.3
(22) Date of filing: 23.10.2017
(51) Int. Cl.: G01N 27/327, C07C 71/00, C07C 309/73, C07D 347/00

(54) **OXIDIZING AGENT FOR ELECTRON MEDIATOR**

(30) Priority: 24.10.2016 JP 2016208024
(71) Applicant: PHC Holdings Corporation, Tokyo 105-8433 (JP)
(72) Inventor: HANEDA, Keigo, Toon-shi, Ehime 791-0395 (JP); EDAGAWA, Kazuaki, Toon-shi, Ehime 791-0395 (JP)
(74) Representative: Vigand, Philippe
(86) International application number: PCT/JP2017/038134
(87) International publication number: WO 2018/079465

(57) **Abstract**

Provided are an oxidizing agent for an electron mediator, said oxidizing agent including a trivalent iodobenzene compound; an analyte measurement reagent including the oxidizing agent; a sensor supporting the oxidizing agent; and a method for the electrochemical measurement of an analyte using the oxidizing agent.

## Description

### Technical Field

The present disclosure relates to a method of electrochemically measuring an analyte via an electron mediator, and an oxidant for an electron mediator for use in a biosensor using the electron mediator.

### Background Art

A method of electrochemically measuring an analyte in a sample by use of an electron mediator is broadly known. The electron mediator is a redox substance that mediates electron transfer, and shoulders responsibility for transfer of an electron produced by oxidation-reduction reaction of an analyte in a biosensor.

For example, Patent Literature 1 discloses a biosensor for measuring total hemoglobin using potassium ferricyanide as an electron mediator. Specifically, Patent Literature 1 discloses that potassium ferricyanide oxidizes hemoglobin iron (II) while potassium ferricyanide is reduced to potassium ferrocyanide. At the same time, an electrode system comprising a working electrode and a counter electrode is used to obtain an electrochemical signal from the potassium ferrocyanide, and a total hemoglobin amount is determined based on the signal.

Patent Literature 2 discloses a biosensor for measuring glucose concentration in blood (blood sugar level). Specifically, Patent Literature 2 discloses that potassium ferricyanide is used as an electron mediator, and when glucose and potassium ferricyanide are reacted in the presence of glucose dehydrogenase, the glucose is reduced by the catalytic action of glucose dehydrogenase while the potassium ferricyanide is reduced to potassium ferrocyanide. At the same time, an electrode system comprising a working electrode and a counter electrode is used to obtain an electrochemical signal from the potassium ferrocyanide, and the glucose amount is determined based on the signal.

However, development of an electrochemical technique with increased measurement accuracy is still needed.

### Citation List

### Patent Literatures

Patent Literature 1: Japanese Patent No. 5812957
Patent Literature 2: Japanese Patent No. 5325574

### Summary of Invention

### Problems to be solved by the Invention

The present inventors found out a technical problem that an electron mediator is reduced depending on storage conditions of the electron mediator, independently of oxidation reaction of an analyte in electrochemical measurement process or in production process of an electrochemical sensor using electrochemical measurement technique. For example, in the case of using potassium ferricyanide as an electron mediator, it was found that when a solution of potassium ferricyanide was added dropwise to an electrode system and dried thereon, a part of the potassium ferricyanide was reduced to potassium ferrocyanide by influence of moisture in the air and light. Further, it was found that when a solution containing a reagent such as a high hygroscopic buffer component and potassium ferricyanide was added dropwise to an electrode system and dried thereon, a part of the potassium ferricyanide was reduced to potassium ferrocyanide depending on the storage conditions. Such unintended reduction of an electron mediator affects the measurement value of an analyte, resulting in decreased measurement accuracy.

### Solutions to the Problems

Thus the present inventors intensively studied, and as a result, surprisingly found that use of an oxidant comprising a trivalent iodobenzene compound could solve the above-described problem.

The present disclosure provides the following aspects:
[1] an oxidant for an electron mediator, comprising a trivalent iodobenzene compound,
[2] the oxidant according to [1], wherein the trivalent iodobenzene compound is represented by any of formulae I to III: 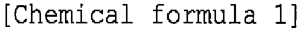 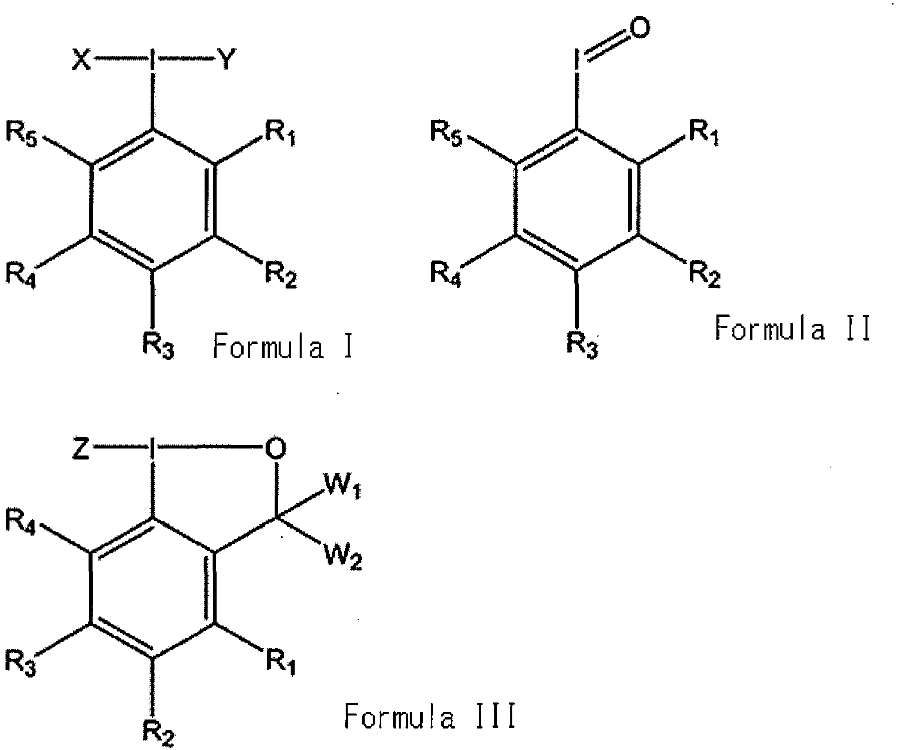 wherein:
   R₁ to R₅ are independently a hydrogen atom, halogen, an amino group, a nitro group, a hydroxyl group, a carboxyl group, an acetyl group, or an optionally substituted hydrocarbon group,
   X and Y are independently a hydroxyl group, an aklylcarbonyloxy group, an alkylsulfonyloxy group, an alkylbenzenesulfonyloxy group, or an optionally substituted hydrocarbon group,
   Z is halogen, a hydroxyl group, an alkylcarbonyloxy group, an alkylperoxy group, or an optionally substituted hydrocarbon group,
   W₁ and W₂ are independently a methyl group, or W₁ and W₂ together form oxo (=O),
[3] the oxidant according to [2], wherein in formula I, R₁ to R₅ are independently a hydrogen atom, halogen, a nitro group, or a linear or branched, saturated or unsaturated, optionally substituted C₁₋₄ hydrocarbon group,
   X and Y are independently a hydroxyl group, an acetoxy group, a trifluoroacetoxy group, a tosyloxy group, a methanesulfonyloxy group, a trifluoromethanesulfonyloxy group, or a linear saturated halogenated C₁₋₁₀ hydrocarbon group;
   in formula II, R₁ to R₅ are independently a hydrogen atom, halogen, a nitro group, a carboxyl group, or a linear or branched, saturated or unsaturated, optionally substituted C₁₋₄ hydrocarbon group; and
   in formula III, R₁ to R₄ are independently a hydrogen atom, halogen, a nitro group, or a linear or branched, saturated or unsaturated, optionally substituted C₁₋₄ hydrocarbon group,
   Z is halogen, a hydroxyl group, an acetoxy group, a trifluoroacetoxy group, or a linear or branched C₁₋₆ alkylperoxy group,
   W₁ and W₂ are independently a methyl group, or W₁ and W₂ together form oxo (=O),
[4] the oxidant according to [1], wherein the trivalent iodobenzene compound is selected from the group consisting of iodosobenzene, 2,4,6-trimethyl(diacetoxyiodo)benzene, [bis(trifluoroacetoxy)iodo]pentafluorobenzene, [hydroxy(tosyloxy)iodo]benzene, 2-iodosobenzoic acid, 1-fluoro-3,3-dimethyl-1,2-benziodoxol, and [hydroxy(methanesulfonyloxy)iodo]benzene,
[5] the oxidant according to any one of [1]-[4], wherein the electron mediator is selected from the group consisting of a metal complex, a quinone compound, a phenazine compound, a viologen compound, a phenothiazine compound, and a phenol compound,
[6] the oxidant according to [5], wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol,
[7] a reagent for measurement of an analyte, containing an electron mediator, and the oxidant according to any one of [1]-[4],
[8] the reagent for measurement of an analyte according to [7], further containing an enzyme that catalyzes oxidation reaction,
[9] the reagent for measurement of an analyte according to [7] or [8], wherein the electron mediator is selected from the group consisting of a metal complex, a quinone compound, a phenazine compound, a viologen compound, a phenothiazine compound, and a phenol compound,
[10] the reagent for measurement of an analyte according to [9], wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol,
[11] a sensor comprising a working electrode, a counter electrode, and a reagent layer, wherein the reagent layer contains an electron mediator and the oxidant according to any one of [1]-[4],
[12] the sensor according to [11], wherein the reagent layer further contains an enzyme that catalyzes oxidation reaction,
[13] the sensor according to [11] or [12], wherein the electron mediator is selected from the group consisting of a metal complex, a quinone compound, a phenazine compound, a viologen compound, a phenothiazine compound, and a phenol compound,
[14] the sensor according to [13], wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol,
[15] a method of electrochemically measuring an analyte, the method comprising:
   (A) bringing a sample containing the analyte into contact with a reagent containing an electron mediator and the oxidant according to any one of [1]-[4], thereby the analyte is oxidized while the electron mediator is reduced, and
   (B) using an electrode system comprising at least a working electrode and a counter electrode to obtain an electrochemical signal from the reduced electron mediator,
[16] the method according to [15], wherein in step (A), the analyte is oxidized by the electron mediator while the electron mediator is reduced,
[17] the method according to [15], wherein in step (A), the reagent further contains an enzyme that catalyzes oxidation reaction, and the analyte is oxidized by the enzyme while the electron mediator is reduced,
[18] the method according to any one of [15]-[17], wherein the electron mediator is selected from the group consisting of a metal complex, a quinone compound, a phenazine compound, a viologen compound, a phenothiazine compound, and a phenol compound,
[19] the method according to [18], wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol,
[20] a use of the oxidant according to any one of [1]-[4] for decreasing degradation of an electron mediator,
[21] the use according to [20], wherein the electron mediator is selected from the group consisting of a metal complex, a quinone compound, a phenazine compound, a viologen compound, a phenothiazine compound, and a phenol compound,
[22] the use according to [21], wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol,
[23] a method of preventing degradation of an electron mediator, the method comprising using the oxidant according to any one of [1]-[4],
[24] the method according to [23], wherein the electron mediator is selected from the group consisting of a metal complex, a quinone compound, a phenazine compound, a viologen compound, a phenothiazine compound, and a phenol compound, and
[25] the method according to [24], wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol.

### Effects of the Invention

The accuracy of electrochemical measurement of an analyte is increased by using the oxidant disclosed herein.

### Brief Description of Drawings

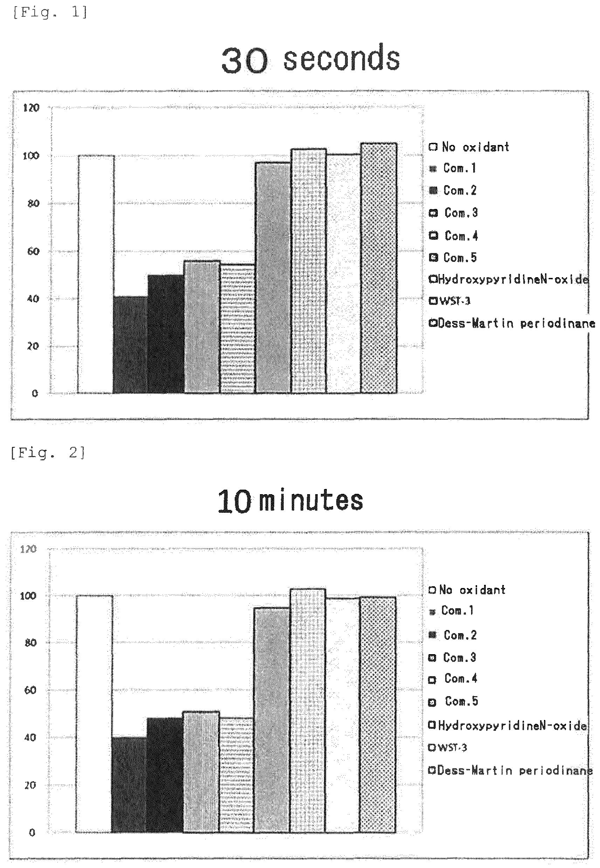

Figure 1 shows the oxidizing action of the oxidant for an electron mediator as disclosed herein on a reduced electron mediator. Current values obtained by use of no oxidant, Compound 1, Compound 2, Compound 3, Compound 4, Compound 5, hydroxypyridine N-oxide, WST-3, and Dess-Martin periodinane are shown from left to right. Each current value is represented as a relative value (%) to a current value obtained by using no oxidant.
Figure 2 shows the oxidizing action of the oxidant for an electron mediator as disclosed herein on a reduced electron mediator. Current values obtained by use of no oxidant, Compound 1, Compound 2, Compound 3, Compound 4, Compound 5, hydroxypyridine N-oxide, WST-3, and Dess-Martin periodinane are shown from left to right. Each current value is represented as a relative value (%) to a current value obtained by using no oxidant.

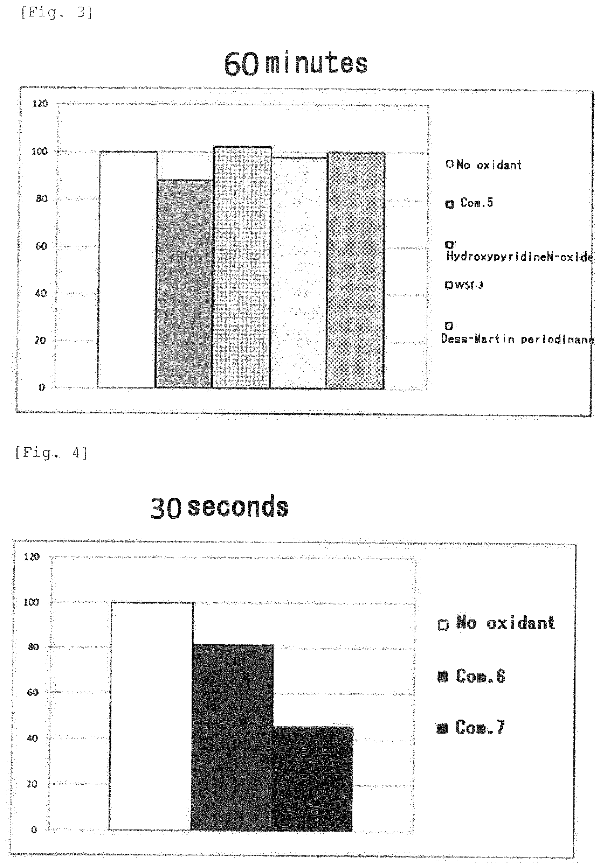

Figure 3 shows the oxidizing action of the oxidant for an electron mediator as disclosed herein on a reduced electron mediator. Current values obtained by use of no oxidant, compound 5, hydroxypyridine N-oxide, WST-3, and Dess-Martin periodinane are shown from left to right. Each current value is represented as a relative value (%) to a current value obtained by using no oxidant.
Figure 4 shows the oxidizing action of the oxidant for an electron mediator as disclosed herein on a reduced electron mediator. Current values obtained by use of no oxidant, Compound 6, and Compound 7 are shown from left to right. Each current value is represented as a relative value (%) to a current value obtained by using no oxidant.

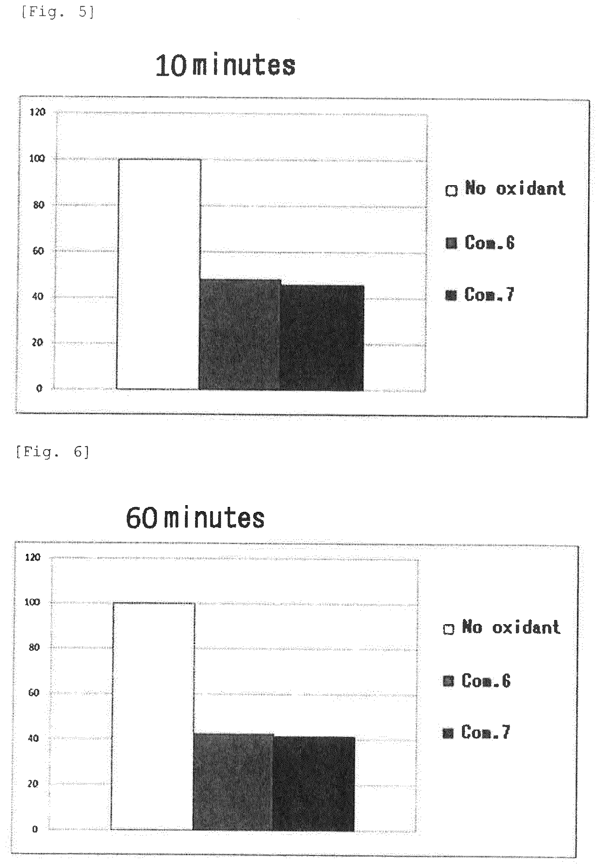

Figure 5 shows the oxidizing action of the oxidant for an electron mediator as disclosed herein on a reduced electron mediator. Current values obtained by use of no oxidant, Compound 6, and Compound 7 are shown from left to right. Each current value is represented as a relative value (%) to a current value obtained by using no oxidant.
Figure 6 shows the oxidizing action of the oxidant for an electron mediator as disclosed herein on reduced electron mediator. Current values obtained by use of no oxidant, Compound 6, and Compound 7 are shown from left to right. Each current value is represented as a relative value (%) to a current value obtained by using no oxidant.

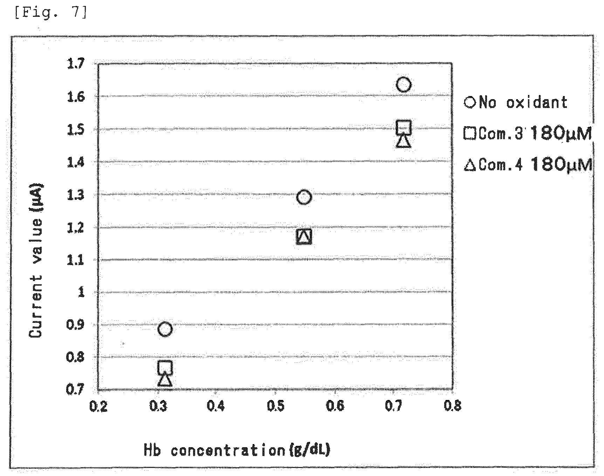

Figure 7 shows measurement results of hemoglobin using sensors loaded with the oxidant for an electron mediator as disclosed herein.

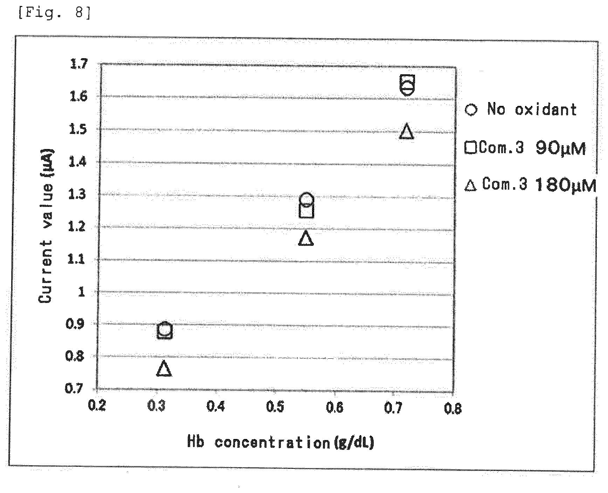

Figure 8 shows measurement results of hemoglobin using sensors loaded with the oxidant for an electron mediator as disclosed herein at different concentrations.

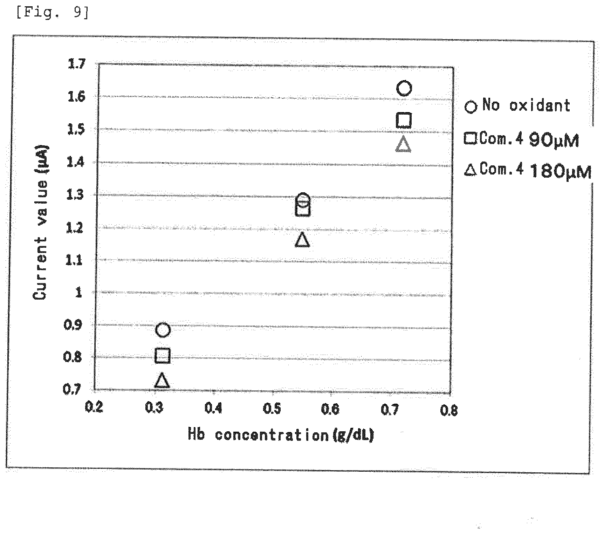

Figure 9 shows measurement results of hemoglobin using sensors loaded with the oxidant for an electron mediator as disclosed herein at different concentrations.

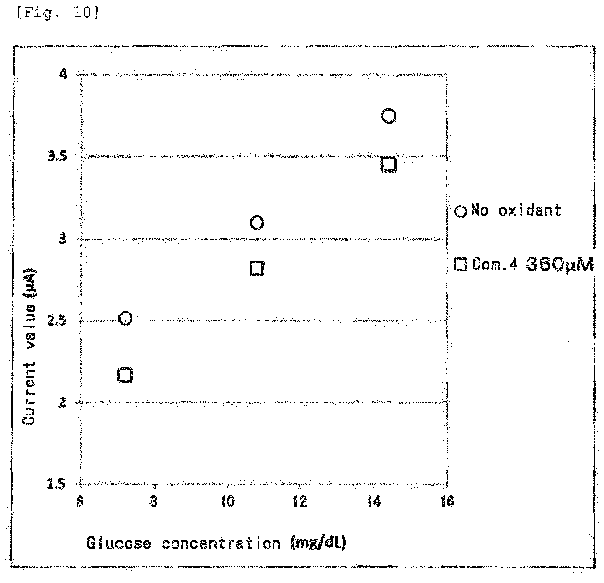

Figure 10 shows measurement results of glucose using a sensor loaded with the oxidant for an electron mediator as disclosed herein.

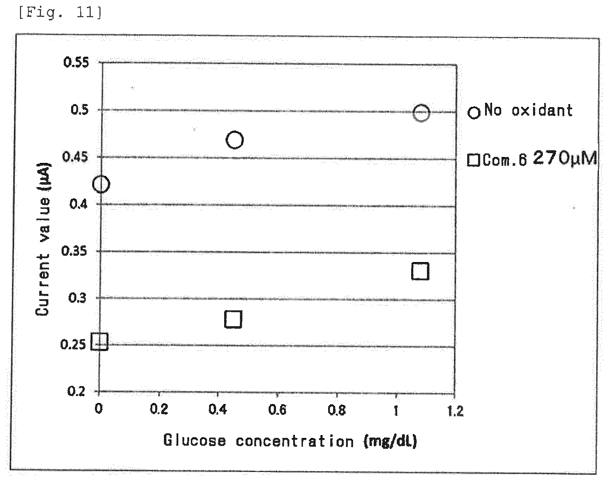

Figure 11 shows measurement results of glucose using a sensor loaded with the oxidant for an electron mediator as disclosed herein.

### Description of Embodiments

### 1. Oxidant for electron mediator as disclosed herein

The oxidant for an electron mediator as disclosed herein (hereinafter, also referred to as "the oxidant disclosed herein") is an oxidant for oxidizing an electron mediator. The oxidant disclosed herein is particularly used for oxidizing an electron mediator that has been reduced independently of oxidation reaction of an analyte in electrochemical measurement of the analyte using an electron mediator or in a process of production of an electrochemical sensor using the electrochemical measurement technique. As used herein, the term "analyte" means a substance to be measured.

An electron mediator was found to be reduced depending on storage conditions including moisture in the air, light, components of a buffer for dissolving the electron mediator (in particular, high hygroscopic buffer components including many phosphates such as disodium hydrogenphosphate, and sodium dihydrogen phosphate, citric acid, etc.) and the like. The degradation of an electron mediator as described above can be decreased or prevented by making the oxidant disclosed herein coexist with the electron mediator.

Specifically, the oxidant disclosed herein comprises a trivalent iodobenzene compound. The oxidant disclosed herein may be a trivalent iodobenzene compound itself, or may comprise a trivalent iodobenzene compound and, as necessary, an additional component that does not adversely affect the oxidizing action of the trivalent iodobenzene compound, such as water, a solvent, a buffer, protein, a polymeric material, sugar, a salt, or a surfactant. The oxidant disclosed herein may comprise two or more kinds of trivalent iodobenzene compounds.

Examples of the trivalent iodobenzene compound used in the present disclosure include, but not limited to, compounds represented by formulae I to III: 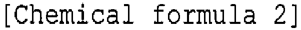 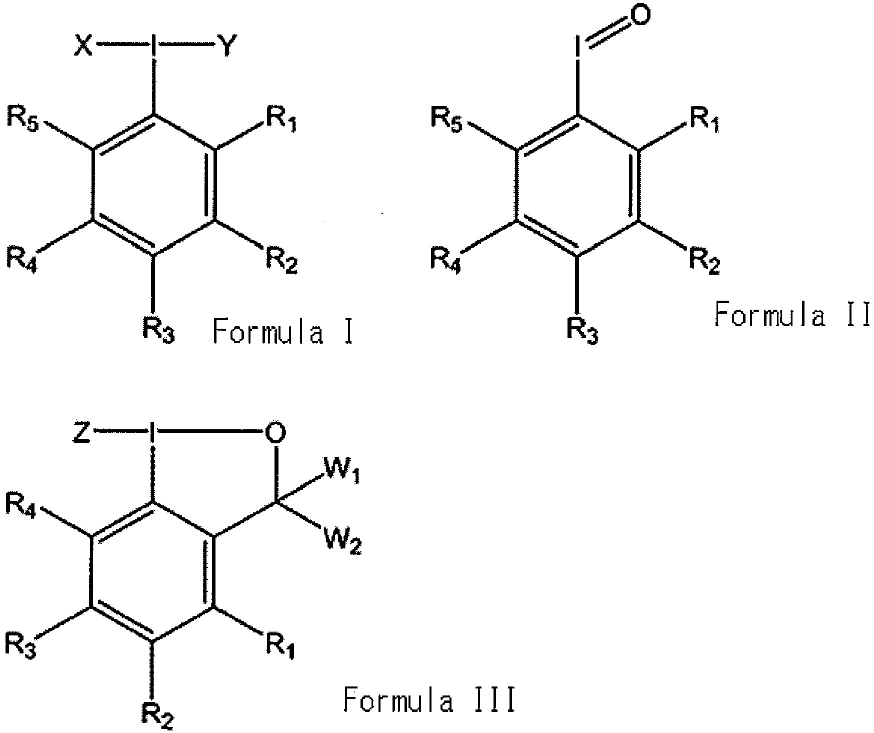 wherein:
R₁ to R₅ are independently a hydrogen atom, halogen, an amino group, a nitro group, a hydroxyl group, a carboxyl group, an acetyl group, or an optionally substituted hydrocarbon group,
X and Y are independently a hydroxyl group, an aklylcarbonyloxy group, an alkylsulfonyloxy group, an alkylbenzenesulfonyloxy group, or an optionally substituted hydrocarbon group,
Z is halogen, a hydroxyl group, an alkylcarbonyloxy group, an alkylperoxy group, or an optionally substituted hydrocarbon group,
W₁ and W₂ are independently a methyl group, or W₁ and W₂ together form oxo (=O).

As used herein, examples of the halogen include bromine, chlorine, iodine, and fluorine. Preferably bromine or fluorine is used.

As used herein, the hydrocarbon group includes a saturated or unsaturated, linear, branched or cyclic hydrocarbon group. Examples of the hydrocarbon group include linear, branched or cyclic hydrocarbon groups containing 1 to 10 carbon atoms. Examples of a substituent that the hydrocarbon group optionally has include at least one substituent selected from the group consisting of halogen, an amino group, a hydroxyl group, a carboxyl group, and an acetyl group. It is preferable that the hydrocarbon group is substituted with halogen or unsubstituted. The number of a substituent(s) that the hydrocarbon group optionally has is not particularly limited. A part of the hydrogen atoms that the hydrocarbon group contains may be substituted with a substituent(s), or all of the hydrogen atoms that the hydrocarbon group contains may be substituted with substituents.

As used herein, alkyl may be substituted with halogen, and a part or all of the hydrogen atoms that the alkyl contains may be substituted with halogen. Examples of alkyl include linear or branched alkyl having one to six carbons, preferably one to four carbons.

As used herein, preferable examples of the trivalent iodobenzene compound include compounds represented by formula I, formula II and formula III, wherein R₁ to R₅ are independently a hydrogen atom, halogen, an amino group, a nitro group, a hydroxyl group, a carboxyl group, an acetyl group, or a linear or branched, saturated or unsaturated optionally substituted C₁₋₄ hydrocarbon group;
X and Y are independently a hydroxyl group, an acetoxy group, a trifluoroacetoxy group, a tosyloxy group, a methanesulfonyloxy group, a trifluoromethanesulfonyloxy group, or a linear or branched, saturated or unsaturated, optionally substituted C₁₋₁₀ hydrocarbon group;
Z is halogen, a hydroxyl group, an acetoxy group, a trifluoroacetoxy group, a linear or branched C₁₋₆ alkylperoxy group, or a linear or branched, saturated or unsaturated, optionally substituted C₁₋₄ hydrocarbon group;
W₁ and W₂ are independently a methyl group, or W₁ and W₂ together form oxo (=O).

In formula I, R₁ to R₅ are, more preferably, independently a hydrogen atom, halogen, a nitro group, or a linear or branched, saturated or unsaturated, optionally substituted C₁₋₄ hydrocarbon group, and further preferably, R₁ to R₅ are independently a hydrogen atom, halogen, a nitro group, or a saturated or unsaturated, unsubstituted C₁₋₂ hydrocarbon group; X and Y are, more preferably, independently a hydroxyl group, an acetoxy group, a trifluoroacetoxy group, a tosyloxy group, a methanesulfonyloxy group, a trifluoromethanesulfonyloxy group, or a linear saturated halogenated C₁₋₁₀ hydrocarbon group, and further preferably, X and Y are the same and are an acetoxy group, a trifluoroacetoxy group, a tosyloxy group, a methanesulfonyloxy group, or a trifluoromethanesulfonyloxy group, or one of X and Y is a hydroxyl group or a linear saturated halogenated C₁₋₁₀ hydrocarbon group and the other of X and Y is an acetoxy group, a trifluoroacetoxy group, a tosyloxy group, a methanesulfonyloxy group, or a trifluoromethanesulfonyloxy group. Still further preferably, in formula I, all of R₁ to R₅ are the same; R₁, R₃ and R₅ are the same and are any one of the above-mentioned substituents other than a hydrogen atom, and R₂ and R₄ are the same and are a hydrogen atom; or R₃ is any one of the above-mentioned substituents other than a hydrogen atom, and R₁, R₂, R₄ and R₅ are the same and are a hydrogen atom. When all of R₁ to R₅ are the same, they are preferably hydrogen or halogen. Further preferably, in formula I, X and y are the same and are an acetoxy group or a trifluoroacetoxy group; or X is a hydroxyl group and Y is a tosyloxy group or a methanesulfonyloxy group.

In formula I, when R₃ is a vinyl group, the compound represented by formula I may take a polymer form as polystyrene. Such a trivalent iodobenzene compound in a polymer form is also included in the oxidant disclosed herein.

In formula II, more preferably, R₁ to R₅ are independently a hydrogen atom, halogen, a nitro group, a carboxyl group, or a linear or branched, saturated or unsaturated, optionally substituted C₁₋₄ hydrocarbon group. Further preferably, in formula II, R₁ to R₅ are independently a hydrogen atom, halogen, a nitro group, a carboxyl group, or a saturated or unsaturated unsubstituted C₁₋₂ hydrocarbon group.

In formula II, when iodosobenzene is substituted with a carboxyl group, the carboxyl group is preferably at an ortho position. In formula II, when iodosobenzene is ortho-substituted with a carboxyl group, the iodosobenzene compound may be cyclized to take a tautomeric form represented by formula III.

The iodosobenzene compound represented by formula II wherein R₁ and R₅ are not a carboxyl group is usually regarded as being in a form of a polymer consisting of a I-O-I-O chain. Such a iodosobenzene compound in a polymer form is also included in the oxidant disclosed herein.

In formula III, more preferably, R₁ to R₄ are independently a hydrogen atom, halogen, a nitro group, or a linear or branched, saturated or unsaturated, optionally substituted C₁₋₄ hydrocarbon group, and further preferably, R₁ to R₄ are independently a hydrogen atom, halogen, a nitro group, or a saturated or unsaturated, unsubstituted C₁₋₂ hydrocarbon group; more preferably, Z is halogen, a hydroxyl group, an acetoxy group, a trifluoroacetoxy group, or a linear or branched C₁₋₆ alkylperoxy group, and further preferably, Z is fluorine, a hydroxyl group, an acetoxy group, or a linear or branched C₁₋₄ alkylperoxy group; W₁ and W₂ are independently a methyl group, or W₁ and W₂ together form oxo (=O). Further preferably, in formula III, R₂ is a hydrogen atom, halogen or a nitro group, and R₁, R₃ and R₄ are the same and are a hydrogen atom.

Specific examples of the trivalent iodobenzene compound used as the oxidant herein include, but not limited to, iodosobenzene, 2,4,6-trimethyl(diacetoxyiodo)benzene, [bis(trifluoroacetoxy)iodo]pentafluorobenzene, [hydroxy(tosyloxy)iodo]benzene, 2-iodosobenzoic acid [also referred to as 1-hydroxy-1,2-benziodoxol-3(1H)-one], 1-acetoxy-5-bromo-1,2-benziodoxol-3(1H)-one, 1-acetoxy-5-nitro-1,2-benziodoxol-3(1H)-one, [bis(trifluoroacetoxy)iodo]benzene, 4-nitro(diacetoxyiodo)benzene, 1-(tert-butylperoxy)-1,2-benziodoxol-3(1H)-one, (diacetoxyiodo)benzene, [hydroxy(methanesulfonyloxy)iodo]benzene, poly[4-(diacetoxyiodo)styrene], 1-fluoro-3,3-dimethyl-1,2-benziodoxol, (perfluoropropyl)phenyliodonium trifluoromethanesulfonate, (perfluorohexyl)phenyliodonium trifluoromethanesulfonate, and (perfluoro-n-octyl)phenyliodonium trifluoromethanesulfonate.

The electron mediator to be oxidized by the oxidant disclosed herein is not particularly limited, and may be any electron mediator that is able to be used in electrochemical measurement. Examples of the electron mediator include, but not limited to, a metal complex (for example, an osmium complex, a ruthenium complex, a ferric complex, etc.), a quinone compound (for example, benzoquinone, naphthoquinone, phenanthrenequinone, phenanthrolinequinone, anthraquinone, and their derivatives), a phenazine compound, a viologen compound, a phenothiazine compound, and a phenol compound. For example, one or more compounds selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol are used as the electron mediator. Examples of the above-mentioned salt include, but not limited to, a sodium salt, a potassium salt, a calcium salt, a magnesium salt, and a lithium salt.

### 2. Measurement method as disclosed herein

The oxidant disclosed herein can be used in electrochemical measurement of an analyte by being made to coexist with an electron mediator. The coexistence of the oxidant disclosed herein with the electron mediator in electrochemical measurement of an analyte can lead to oxidation of the electron mediator that has been reduced depending on storage conditions including moisture in the air, light, components in a solution and the like, and thereby a decrease in the measurement accuracy caused by the reduction of electron mediator that is independent of the analyte is prevented and an accurate measurement value of the analyte can be obtained. Thus, use of the oxidant disclosed herein in electrochemical measurement of an analyte results in an increase in the measurement accuracy, and thereby a more accurate measurement value of the analyte can be obtained.

A method of electrochemically measuring an analyte which is characterized by using the oxidant disclosed herein (hereinafter, also referred to as "the measurement method disclosed herein") comprises the following steps:
(A) bringing a sample containing the analyte into contact with a reagent containing an electron mediator and the oxidant disclosed herein, thereby the analyte is oxidized while the electron mediator is reduced, and
(B) using an electrode system comprising at least a working electrode and a counter electrode to obtain an electrochemical signal from the reduced electron mediator. As used herein, the term "measurement" includes "quantitative" measurement and "qualitative" measurement.

Examples of the analyte to be measured by the measurement method disclosed herein include, but not limited to, hemoglobin, glucose, ketone body (3-hydroxy butyric acid), lactic acid, glycated hemoglobin, and glycated albumin.

The sample to be subjected to the measurement method disclosed herein may be any sample containing the analyte, and is not particularly limited. Examples of the sample include a blood sample such as whole blood, plasma, a serum, a blood cell, diluted blood, etc. (for example, a diluted blood sample from human), a biological sample such as urine, spinal fluid, sweat, tears, saliva, skin, mucous membrane, hair, etc., various foods or extract thereof, and beverage such as alcohol and juice. The biological sample may not be derived from human. The blood sample may be subjected to hemolysis treatment. The hemolysis treatment may be carried out by a conventional method.

The electron mediator used in the measurement method disclosed herein is not particularly limited, and can be determined as appropriate depending on the kind of the analyte and measurement conditions. For example, the above-mentioned electron mediators can be used.

In step (A) of the measurement method disclosed herein, the analyte in the sample is oxidized by the electron mediator, or is made to coexist with an enzyme that catalyzes oxidation reaction and oxidized by the enzyme. In the latter case, the reagent containing an electron mediator and the oxidant disclosed herein further contains the enzyme that catalyzes oxidation reaction. The enzyme can be determined as appropriate depending on the kind of the analyte. Examples of the enzyme include oxidoreductase, such as oxidase and dehydrogenase. Specific examples thereof include, but not limited to, glucose dehydrogenase, glucose oxidase, 3-hydroxybutyrate dehydrogenase, lactate dehydrogenase, lactate oxidase, cholesterol oxidase, and fructosyl peptide oxidase. The enzyme may be dependent on a coenzyme. Examples of the coenzyme include, but not limited to, pyrroloquinoline quinone (PQQ), and flavin adenine dinucleotide (FAD).

For example, when the analyte is hemoglobin, the ferrous iron of hemoglobin is oxidized by an electron mediator sensitive to hemoglobin such as potassium ferricyanide, and at the same time, the electron mediator is reduced. For example, when the analyte is glucose and glucose dehydrogenase is used as the enzyme, the glucose dehydrogenase reacts with the glucose to generate gluconolactone and electrons, and an electron mediator is reduced by the electrons. For example, when the analyte is lactic acid and lactate oxidase is used as the enzyme, the lactate oxidase reacts with the lactic acid to generate pyruvic acid and electrons, and an electron mediator is reduced by the electrons. For example, when the analyte is 3-hydroxybutyric acid and 3-hydroxybutyrate dehydrogenase is used as the enzyme, the 3-hydroxybutyrate dehydrogenase reacts with the 3-hydroxybutyric acid to generate acetoacetic acid and electrons, and nicotinamide adenine dinucleotide is reduced by the electrons. Then, the reduced nicotinamide adenine dinucleotide reduces an electron mediator directly or via diaphorase.

The amount of the oxidant disclosed herein used in the measurement method disclosed herein is not particularly limited, and it is determined as appropriate depending on the sample to be measured, the kind or amount of the analyte or electron mediator, the kind of the oxidant, and other measurement conditions. Examples of the reaction final concentration of the oxidant disclosed herein include, but not limited to, ranges of about 10 µM to about 5 mM, about 30 µM to about 1 mM, and about 50 µM to about 500 µM.

The amounts of the electron mediator and the enzyme used in the measurement method disclosed herein are not particularly limited, and they may be determined as appropriate depending on the sample to be measured, the kind or amount of the analyte, oxidant or electron mediator, the kind of the enzyme, and other measurement conditions.

For the purpose of stabilization and the like of the reagent, the reagent may further contain an additional component that does not adversely affect the action of the electron mediator, oxidant and enzyme, such as a buffer, a surfactant, a polymeric material, organic acid, protein, sugar, or a salt, as necessary.

The electrode system used in the measurement method disclosed herein comprises at least a working electrode and a counter electrode. The electrode system may further comprise a reference electrode. The material of the electrode is not particularly limited, and may be any material usually used in electrochemical measurement, such as platinum, gold, silver, palladium, carbon, iridium, or iridium oxide. Preferably, gold, platinum, palladium or carbon is used as the electrode material. The electrode system may be loaded with a reagent containing an electron mediator and the oxidant disclosed herein, or a reagent containing an electron mediator, the oxidant disclosed herein and an enzyme that catalyzes oxidation reaction. The reagent may be preferably loaded into a reagent layer formed on the electrodes.

In the measurement method disclosed herein, the contact of the sample containing the analyte with the reagent containing an electron mediator and the oxidant disclosed herein is preferably carried out on electrodes. For example, the contact may be carried out by applying the sample to electrodes loaded with the reagent or by immersing the electrodes in the sample.

In the measurement method disclosed herein, the electrochemical signal reflects the amount of the analyte. Examples of the electrochemical signal include current, the quantity of electric charge, electric potential, and impedance. In the measurement method disclosed herein, the electrochemical signal can be acquired by applying a predetermined voltage across a working electrode and a counter electrode or, in the case of three-electrode system, applying a predetermined voltage based on a reference electrode to a working electrode to oxidize a reduced electron mediator. Specifically, oxidation reaction of a reduced electron mediator is induced by the applied voltage as described above, and then, the value of an electric current flowing along the working electrode or the quantity of the electric charge is detected.

The "predetermined voltage" means an electric potential at which a reduced electron mediator is oxidized. The predetermined voltage is determined as appropriate depending on various conditions such as the electrode material and pH. Examples of the predetermined voltage when the reduced electron mediator is potassium ferrocyanide (potassium hexacyanoferrate(II)) include, but not limited to, about 0.3 V to 0.6 V in the case of three-electrode system [a working electrode (for example, platinum or palladium), a counter electrode, and a reference electrode (for example, silver/silver chloride (saturated potassium chloride) electrode)], and about 0.2 V to 0.4 V in the case of two-electrode system (a working electrode and a counter electrode).

### 3. Reagent for measurement as disclosed herein

The present disclosure provides a reagent for analyte measurement containing an electron mediator and the oxidant disclosed herein (hereinafter, also referred to as "the reagent for measurement disclosed herein"). The reagent for measurement disclosed herein may further contain an enzyme that catalyzes oxidation reaction. The enzyme is an enzyme that catalyzes the oxidation reaction of an analyte. The reagent for measurement disclosed herein is used for electrochemical measurement of an analyte. The reagent for measurement disclosed herein is preferably used in the measurement method disclosed herein. The electron mediator, analyte and enzyme are as described above.

The reagent for measurement disclosed herein may further contain, as necessary, an additional component that does not adversely affect the action of the electron mediator, oxidant and enzyme, such as a buffer, a surfactant, a polymeric material, organic acid, protein, sugar, or a salt.

### 4. Sensor as disclosed herein

The present disclosure provides a sensor for measuring an analyte in a sample, which comprises an electrode system containing a working electrode and a counter electrode, and a reagent layer containing an electron mediator and the oxidant disclosed herein (hereinafter, also referred to as "the sensor disclosed herein"). The reagent layer may further contain an enzyme that catalyzes oxidation reaction. The enzyme is an enzyme that catalyzes the oxidation reaction of an analyte. In the sensor disclosed herein, the electrode system may further contain a reference electrode. The sensor disclosed herein is used for electrochemical measurement of an analyte. The sensor disclosed herein is preferably used in the measurement method disclosed herein. The electron mediator, analyte and enzyme as well as the working electrode, counter electrode and reference electrode are as described above. A method of producing the sensor disclosed herein may follow a known method in the art.

In the sensor disclosed herein, the reagent layer is formed near the electrodes or on the electrodes. The reagent layer is preferably formed on the electrodes. The reagent layer may be formed only on the working electrode, or may be formed on both the working electrode and the counter electrode. The electrodes may be formed on an insulating substrate. Examples of the insulating substrate include, but not limited to, resin such as polyethylene terephthalate, vinyl polymer, polyimide, polyester, and styrenics; glass, and ceramics. A method of loading the electron mediator and the oxidant disclosed herein, and, as necessary, the enzyme that catalyzes oxidation reaction into the reagent layer on the electrodes is not particularly limited, and a known method in the art can be used. For example, an ink-jet method may be used. The amounts of the electron mediator, oxidant and enzyme loaded into the reagent layer are not particularly limited, and can be determined as appropriate by a person skilled in the art.

For the purpose of stabilization and the like of reagents, the reagent layer may further contain an additional component that does not adversely affect the action of the electron mediator, oxidant and enzyme, such as a buffer, a surfactant, a polymeric material, organic acid, protein, sugar, or a salt, as necessary.

Hereinafter, the present disclosure is explained in detail based on Examples which the present invention is not limited to.

### EXAMPLE 1

### Example 1:

### Assessment of reactivity of oxidant to reduced electron mediator

This Example demonstrated that the oxidant disclosed herein oxidized a reduced electron mediator.

### (Method)

As the oxidant, iodosobenzene (hereinafter, also referred to as "Compound 1"), 2,4,6-trimethyl(diacetoxyiodo)benzene (hereinafter, also referred to as "Compound 2"), [bis(trifluoroacetoxy)iodo]pentafluorobenzene (hereinafter, also referred to as "Compound 3"), [hydroxy(tosyloxy)iodo]benzene (hereinafter, also referred to as "Compound 4"), 2-iodosobenzoic acid (hereinafter, also referred to as "Compound 5"), 1-fluoro-3,3-dimethyl-1,2-benziodoxol (hereinafter, also referred to as "Compound 6"), and [hydroxy(methanesulfonyloxy)iodo]benzene (hereinafter, also referred to as "Compound 7") (all manufactured by TOKYO KASEI KOGYO CO., LTD.; catalog numbers 10072, 10479, B1616, P1015, 10073, F0957, and P1298, respectively) were used. As the reduced electron mediator, potassium hexacyanoferrate(II) trihydrate (potassium ferrocyanide) (manufactured by Wako Pure Chemical Corporation) was used. As a control oxidant, hydroxypyridine N-oxide (manufactured by Dojindo Laboratories), WST-3 (manufactured by Dojindo Laboratories), and Dess-Martin periodinane (manufactured by TOKYO KASEI KOGYO CO., LTD.) were used.

Of the composition of a measurement solution shown in Table 1, all components other than the oxidant were previously mixed. Then, the oxidant was added to the mixture, and reacted for a predetermined time (30 seconds, 10 minutes, or 60 minutes). Then, the reaction mixture was applied to a sensor comprising a working electrode and a counter electrode that were palladium electrodes. A voltage of 0.4 V was applied to the working electrode and the counter electrode, and 30 seconds after, a current value was measured. As a control, a measurement solution containing no oxidant was prepared, and measured in the same manner as described above.

**[Table 1]**

| Table 1. Composition of measurement solution | |
|---|---|
| | Reaction final concentration |
| Potassium phosphate buffer (pH 6.5) | 50 mM |
| Potassium ferricyanide | 1 mM |
| Potassium ferrocyanide | 150 µM |
| Oxidant | 150 µM |

### (Results)

Results are shown in Figures 1 to 6. In Figures 1 to 6, a current value obtained using the measurement solution containing no oxidant was regarded as 100%, and a relative current value (%) obtained using the measurement solution containing each oxidant is shown. In the figures, "Com.1" to "Com.7" mean "Compound 1" to "Compound 7" respectively.

As is evident from Figures 1 to 6, the current values obtained using Compound 1 to Compound 7 were decreased as compared with the current value obtained using no oxidant. These results show that Compound 1 to Compound 7 oxidized potassium ferrocyanide. When the measurement solutions containing the oxidants (hydroxypyridine N-oxide, WST-3, and Dess-Martin periodinane) other than the oxidant disclosed herein were used, little decrease in the current values was observed as compared with the current values obtained using the measurement solutions containing Compound 1 to Compound 7.

As is evident from Figure 2 and Figure 3, the current value obtained using the measurement solution containing Compound 5 was further decreased by extending the reaction time. Thus it was found that Compound 5 also oxidized potassium ferrocyanide. In contrast, in the case of using the measurement solutions containing other oxidants (hydroxypyridine N-oxide, WST-3, and Dess-Martin periodinane), no remarkable decrease in the current values was observed even when the reaction time was extended.

Thus it was found that Compounds 1 to 7 are superior oxidants for oxidizing a reduced electron mediator.

### EXAMPLE 2

### Example 2: Performance test 1 of sensor comprising the oxidant disclosed herein

An analyte was measured by using a sensor loaded with the oxidant disclosed herein, and the measurement accuracy was assessed.

### (Method)

As the oxidant, [bis(trifluoroacetoxy)iodo]pentafluorobenzene (Compound 3), and [hydroxy(tosyloxy)iodo]benzene (Compound 4) were used. As the electron mediator, potassium hexacyanoferrate(III) trihydrate (potassium ferricyanide) (manufactured by Wako Pure Chemical Corporation) was used. As the analyte, Certified Reference Material for Total Hemoglobin Measurement (purchased from Reference Material Institute for Clinical Chemistry Standards; 25-fold dilutions of JCCRM 912-2L, M ad H were used) was used.

A measurement solution having the composition shown in Table 2 was prepared, and applied to a sensor comprising a working electrode and a counter electrode that were palladium electrodes, loaded with any one of reagents (A) to (D) shown in Table 3. A voltage of 0.2 V was applied to the working electrode and the counter electrode, and 5 seconds after, a current value was measured. As a control, using a sensor loaded with a reagent having the same composition as described above except containing no oxidant, the above-described measurement solution was measured in the same manner as described above. Further, a measurement solution having the same composition as described above except containing no hemoglobin (in other words, containing 5.0%(w/v) of an anion surfactant, 300 mM of potassium glutamate, 30 mM of potassium phosphate buffer (pH 8)) was prepared and applied to the above-described sensor, and then, a blank current value was measured. In tables, "%" all means "%(w/v)". As the anion surfactant, sodium N-dodecanoylsarcosinate was used. As excipient 1, AQUPAANA (partially neutralized polyacrylic acid; manufactured by SUMITOMO SEIKA CHEMICALS CO., LTD.) was used. As excipient 2, STABILIZE QM [(methyl vinyl ether/maleic acid) crosspolymer; manufactured by ISP Japan Ltd.] was used.

**[Table 2]**

| Table 2. Composition of measurement solution | |
|---|---|
| | Reaction final concentration |
| Anion surfactant | 5.0% |
| Potassium glutamate | 300 mM |
| Potassium phosphate buffer | 30 mM (pH 8) |
| Certified reference material for total hemoglobin measurement (JCCRM 912-2L, M, H) | 20-fold dilution |

**[Table 3]**

| Table 3. Composition of reagents | | | |
|---|---|---|---|
| Reagent (A) | | Reagent (B) | |
| | Concentration | | Concentration |
| Potassium ferricyanide | 2% | Potassium ferricyanide | 2% |
| taurine | 1% | taurine | 1% |
| Excipient 1 | 0.06% | Excipient 1 | 0.06% |
| Excipient 2 | 0.02% | Excipient 2 | 0.02% |
| Compound 3 | 90 µM | Compound 4 | 90 µM |
| | | | |

| Reagent (C) | | Reagent (D) | |
|---|---|---|---|
| | Concentration | | Concentration |
| Potassium ferricyanide | 2% | Potassium ferricyanide | 2% |
| taurine | 1% | taurine | 1% |
| Excipient 1 | 0.06% | Excipient 1 | 0.06% |
| Excipient 2 | 0.02% | Excipient 2 | 0.02% |
| Compound 3 | 180 µM | Compound 4 | 180 µM |

### (Results)

Results are shown in Figures 7 to 9. Figure 7 shows current responses to the hemoglobin concentration when the certified reference material for total hemoglobin measurement was measured using the sensor loaded with 180 µM of Compound 3 or Compound 4. Figure 8 and Figure 9 show current responses to the hemoglobin concentration when the certified reference material for total hemoglobin measurement was measured using the sensor loaded with 90 µM or 180 µM of Compound 3 or Compound 4. Table 4 shows equations of linear approximate curves based on each measurement result. Table 5 shows blank current values. Table 6 shows a value (S/N rate) obtained by dividing a current value that was obtained when certified reference material for total hemoglobin measurement JCCRM912-2H was measured by a blank current value.

In the figures, "Com.3" and "Com.4" mean Compound 3 and Compound 4 respectively, and "Hb" means hemoglobin.

**[Table 4]**

| | Linear approximate curve |
|---|---|
| No oxidant | y = 1.83x + 0.31 |
| Compound 3 90 µM | y = 1.89x + 0.27 |
| Compound 3 180 µM | y = 1.81x + 0.19 |
| Compound 4 90 µM | y = 1.81x + 0.25 |
| Compound 4 180 µM | y = 1.81x + 0.17 |

**[Table 5]**

| | Blank current value (µA) |
|---|---|
| No oxidant | 0.281 |
| Compound 3 180 µM | 0.221 |
| Compound 4 180 µM | 0.205 |

**[Table 6]**

| | S/N rate (Current value obtained when JCCRM912-2H was measured / Blank current value) |
|---|---|
| No oxidant | 5.82 |
| Compound 3 180 µM | 6.79 |
| Compound 4 180 µM | 7.16 |

As is evident from Figure 7 and Table 4, when the sensor that was not loaded with the oxidant disclosed herein and the sensor that was loaded with Compound 3 or Compound 4 were compared, the slopes of linear approximate curves were almost the same and there was no difference in change of current response to the hemoglobin concentration. Thus it was found that addition of the oxidant disclosed herein did not decrease the performance of the sensor in measurement after a short period of reaction time. The presence of the oxidant disclosed herein does not affect oxidation of an analyte by an electron mediator and oxidation of the reduced electron mediator induced by the oxidation of the analyte, probably because of their different reaction speeds. On the other hand, in the sensor loaded with Compound 3 or Compound 4, the blank current value was decreased and the S/N rate was increased as compared with the sensor loaded with no oxidant (see Tables 5 and 6). These results show that in the sensor loaded with the oxidant disclosed herein, the measurement accuracy was increased.

Next, whether the effect of the oxidant disclosed herein depends on its concentration was examined. As seen from Figure 8, Figure 9 and Table 4, even when the oxidant disclosed herein was used at different concentrations (90 µM or 180 µM), the slopes of linear approximate curves were almost the same and there was no difference in change of current response to the hemoglobin concentration.

### EXAMPLE 3

### Example 3: Performance test 2 of sensor comprising the oxidant disclosed herein

### (Method)

As the oxidant, [hydroxy(tosyloxy)iodo]benzene (Compound 4) was used. As the electron mediator, sodium 1,2-naphthoquinone-4-sulfonate (manufactured by Wako Pure Chemical Corporation) was used. As the analyte, glucose was used.

A measurement solution was prepared by using phosphate buffered saline (PBS) to dissolve glucose at each concentration of 7.2 mg/dL (400 µM), 10.8 mg/dL (600 µM) or 14.4 mg/dL (800 µM). The measurement solution was applied to a sensor comprising a working electrode and a counter electrode that were palladium electrodes, loaded reagent (E) shown in Table 7. A voltage of 0.25 V was applied to the working electrode and the counter electrode, and 10 seconds after, a current value was measured. As a control, using a sensor loaded with a reagent having the same composition as described above except containing no oxidant, the above-described measurement solution was measured in the same manner as described above. Further, PBS was applied as a measurement solution to the above-described sensor to measure a blank current value. In the table, "%" all means "%(w/v)".

**[Table 7]**

| Table 7. Composition of Reagent (E) | |
|---|---|
| | Concentration |
| sodium 1,2-naphthoquinone-4-sulfonate | 15 mM |
| Sodium phosphate buffer (pH6.5) | 20 mM |
| Carboxymethyl cellulose | 0.2% |
| Glucose dehydrogenase FAD-dependent | 400 U/mL |
| Trehalose | 0.5% |
| Dodecyl maltoside | 0.0075% |
| Compound 4 | 360 µM |

### (Results)

Results are shown in Figure 10. Figure 10 shows current responses to the glucose concentration when glucose was measured using the sensor loaded with Compound 4 and glucose dehydrogenase. Table 8 shows equations of linear approximate curves based on measurement results. Table 9 shows blank current values. Table 10 shows a value (S/N rate) obtained by dividing a current value that was obtained when 14.4 mg/dL of glucose was measured by a blank current value.

In the figure, "Com.4" means "Compound 4".

**[Table 8]**

| | Linear approximate curve |
|---|---|
| No oxidant | y = 0.172x + 1.27 |
| Compound 4 360 µM | y = 0.178x + 0.89 |

**[Table 9]**

| | Blank current value (µA) |
|---|---|
| No oxidant | 1.13 |
| Compound 4 360 µM | 0.71 |

**[Table 10]**

| | S/N rate (Current value obtained when 14.4mg/dL of glucose was measured / Blank current value) |
|---|---|
| No oxidant | 3.31 |
| Compound 4 360 µM | 4.90 |

As is evident from Figure 10 and Table 8, when the sensor that was not loaded with the oxidant disclosed herein and the sensor that was loaded with Compound 4 were compared, the slopes of linear approximate curves were almost the same and there was no difference in change of current response to the glucose concentration. Thus it was found that addition of the oxidant disclosed herein did not decrease the performance of the sensor in measurement after a short period of reaction time. The presence of the oxidant disclosed herein does not affect oxidation of an analyte by an enzyme and oxidation of a reduced electron mediator induced by the oxidation of the analyte, probably because of their different reaction speeds. On the other hand, in the sensor loaded with Compound 4, the blank current value was decreased and the S/N rate was increased as compared with the sensor loaded with no oxidant (see Tables 9 and 10). These results show that in the sensor loaded with the oxidant disclosed herein, measurement accuracy was increased.

### EXAMPLE 4

### Example 4: Performance test 3 of sensor comprising the oxidant disclosed herein

### (Method)

As the oxidant, 1-fluoro-3,3-dimethyl-1,2-benziodoxol (Compound 6) was used. As the electron mediator, 1-hydroxyphenazine (manufactured by TOKYO KASEI KOGYO CO., LTD.) was used in the form of suspension to prepare a reagent. As the analyte, glucose was used.

A measurement solution was prepared by using phosphate buffered saline (PBS) to dissolve glucose at each concentration [0 µM, 25 µM (0.45 mg/dL) or 60 µM (1.08 mg/dL)]. The measurement solution was applied to a sensor comprising a working electrode and a counter electrode that were palladium electrodes, loaded with reagent (F) shown in Table 11. A voltage of 0.25 V was applied across the working electrode and the counter electrode, and 10 seconds after, a current value was measured. As a control, using a sensor loaded with a reagent having the same composition as described above except containing no oxidant, the above-described measurement solution was measured in the same manner as described above. Further, PBS was applied as a measurement solution to the above-described sensor to measure a blank current. In the table, "%" all means "%(w/v)".

**[Table 11]**

| Table 11. Composition of Reagent (F) | |
|---|---|
| | Concentration |
| 1-hydroxyphenazine | 0.067% |
| Sodium phosphate buffer (pH6.5) | 15 mM |
| Carboxymethyl cellulose | 0.2% |
| Glucose dehydrogenase FAD-dependent | 500 U/mL |
| Dodecyl maltoside | 0.0065% |
| Compound 6 | 270 µM |

### (Results)

Results are shown in Figure 11. Figure 11 shows current responses to the glucose concentration when glucose was measured using the sensor loaded with Compound 6 and glucose dehydrogenase. Table 12 shows equations of linear approximate curves based on measurement results. Table 13 shows blank current values. Table 14 shows a value (S/N rate) obtained by dividing a current value that was obtained when 1.08 mg/dL of glucose was measured by a blank current value.

In the figure, "Com.6" means "Compound 6".

**[Table 12]**

| | Linear approximate curve |
|---|---|
| No oxidant | y = 0.0707x + 0.427 |
| Compound 6 270 µM | y = 0.0732x + 0.250 |

**[Table 13]**

| | Blank current value (µA) |
|---|---|
| No oxidant | 0.421 |
| Compound 6 270 µM | 0.253 |

**[Table 14]**

| | S/N rate (Current value obtained when 1.08mg/dL of glucose was measured / Blank current value) |
|---|---|
| No oxidant | 1.19 |
| Compound 6 270 µM | 1.31 |

As is evident from Figure 11 and Table 12, when the sensor that was not loaded with the oxidant disclosed herein and the sensor that was loaded with Compound 6 were compared, the slopes of linear approximate curves were almost the same and there was no difference in change of current response to the glucose concentration. Thus it was found that addition of the oxidant disclosed herein did not decrease the performance of the sensor in measurement after a short period of reaction time. The presence of the oxidant disclosed herein does not affect oxidation of an analyte by an enzyme and oxidation of a reduced electron mediator induced by the oxidation of the analyte, probably because of their different reaction speeds. On the other hand, in the sensor loaded with Compound 6, the blank current value was decreased and the S/N rate was increased as compared with the sensor loaded with no oxidant (see Tables 13 and 14). These results show that in the sensor loaded with the oxidant disclosed herein, measurement accuracy was increased.

### EXAMPLE 5

### Example 5: Performance test 4 of sensor comprising the oxidant disclosed herein (Blank current value)

As the oxidant, 1-fluoro-3,3-dimethyl-1,2-benziodoxol (Compound 6) was used. As the electron mediator, sodium anthraquinone-β-sulfonate (manufactured by Wako Pure Chemical Corporation) was used. As a measurement solution, phosphate buffered saline (PBS) was used.

The measurement solution was applied to a sensor comprising a working electrode and a counter electrode that were palladium electrodes, loaded with reagent (G) shown in Table 15. A voltage of 0.25 V was applied across the working electrode and the counter electrode, and 10 seconds after, a blank current value was measured. As a control, using a sensor loaded with a reagent having the same composition as described above except containing no oxidant, the above-described measurement solution was measured in the same manner as described above. Results are shown in Table 16. In the table, "%" all means "%(w/v)".

**[Table 15]**

| Table 15. Composition of Reagent (G) | |
|---|---|
| | Concentration |
| Sodium anthraquinone-β-sulfonate | 8.2 mM |
| Sodium phosphate buffer (pH6.5) | 15 mM |
| Carboxymethyl cellulose | 0.15% |
| Glucose dehydrogenase FAD-dependent | 500 U/mL |
| Dodecyl maltoside | 0.0065% |
| Compound 6 | 360 µM |

**[Table 16]**

| | Blank current value (µA) |
|---|---|
| No oxidant | 0.1047 |
| Compound 6 360 µM | 0.0652 |

As is evident from Table 16, in the sensor loaded with Compound 6, the blank current value was decreased as compared with the sensor that was not loaded with the oxidant disclosed herein. This result shows that addition of the oxidant disclosed herein decreased the reduced electron mediator that was unintentionally generated when the sensor was produced.

### EXAMPLE 6

### Example 6: Performance test 5 of sensor comprising the oxidant disclosed herein (Blank current value)

As the oxidant, [hydroxy(tosyloxy)iodo]benzene (Compound 4) was used. As the electron mediator, sodium 9,10-phenanthrenequinone-2-sulfonate was used. The sodium 9,10-phenanthrenequinone-2-sulfonate was obtained by sulfonylation of commercially available 9,10-phenanthrenequinone with fuming sulfuric acid, sorting of isomers, and then conversion into the sodium salt. As a measurement solution, phosphate buffered saline (PBS) was used.

The measurement solution was applied to a sensor comprising a working electrode and a counter electrode that were palladium electrodes, loaded with reagent (H) shown in Table 17. A voltage of 0.25 V was applied across the working electrode and the counter electrode, and 10 seconds after, a blank current value was measured. As a control, using a sensor loaded with a reagent having the same composition as described above except containing no oxidant, the above-described measurement solution was measured in the same manner as described above. Results are shown in Table 18. In the table, "%" all means "%(w/v)".

**[Table 17]**

| Table 17. Composition of Reagent (H) | |
|---|---|
| | Concentration |
| Sodium 9,10-phenanthrenequinone-2-sulfonate | 10 mM |
| Sodium phosphate buffer (pH6.5) | 20 mM |
| Carboxymethyl cellulose | 0.2% |
| Trehalose | 0.5% |
| Glucose dehydrogenase FAD-dependent | 400 U/mL |
| Dodecyl maltoside | 0.007% |
| Compound 4 | 360 µM |

**[Table 18]**

| | Blank current value (µA) |
|---|---|
| No oxidant | 0.0869 |
| Compound 4 360 µM | 0.0783 |

As is evident from Table 18, in the sensor loaded with Compound 4, the blank current value was decreased as compared with the sensor that was not loaded with the oxidant disclosed herein. This result shows that addition of the oxidant disclosed herein decreased the reduced electron mediator that was unintentionally generated when the sensor was produced.

### Industrial Applicability

The oxidant for an electron mediator as disclosed herein can be used in electrochemical measurement of various analytes, and increase the measurement accuracy.

## Claims

1. An oxidant for an electron mediator, comprising a trivalent iodobenzene compound.

2. The oxidant according to claim 1, wherein the trivalent iodobenzene compound is represented by any of formulae I to III: 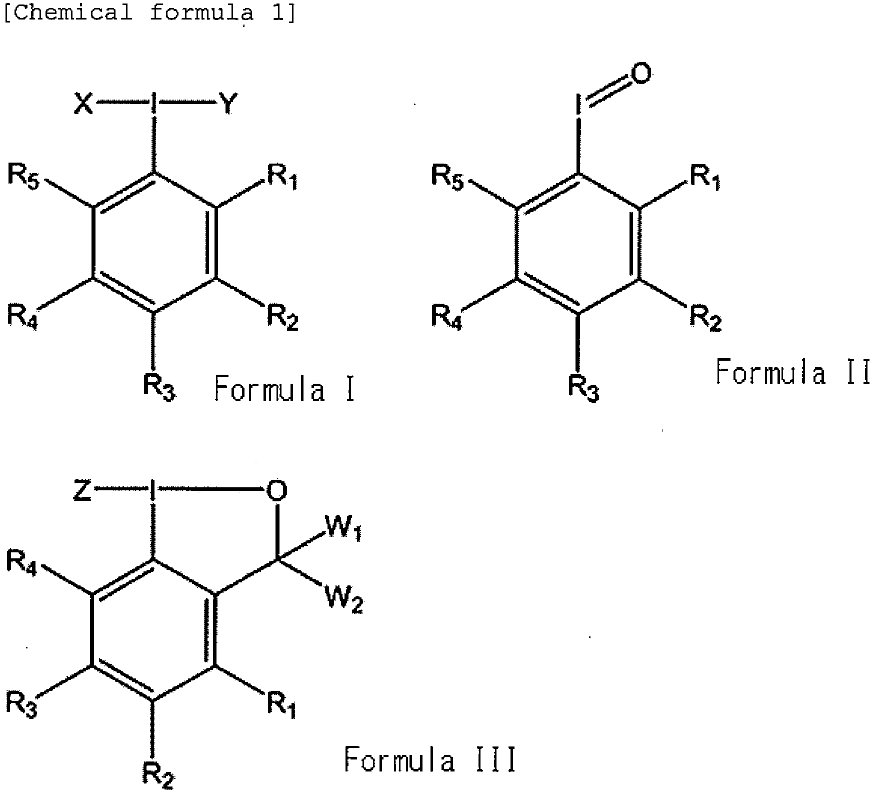 wherein:
R₁ to R₅ are independently a hydrogen atom, halogen, an amino group, a nitro group, a hydroxyl group, a carboxyl group, an acetyl group, or an optionally substituted hydrocarbon group,
X and Y are independently a hydroxyl group, an aklylcarbonyloxy group, an alkylsulfonyloxy group, an alkylbenzenesulfonyloxy group, or an optionally substituted hydrocarbon group,
Z is halogen, a hydroxyl group, an alkylcarbonyloxy group, an alkylperoxy group, or an optionally substituted hydrocarbon group,
W₁ and W₂ are independently a methyl group, or W₁ and W₂ together form oxo (=O).

3. The oxidant according to claim 2, wherein in formula I, R₁ to R₅ are independently a hydrogen atom, halogen, a nitro group, or a linear or branched, saturated or unsaturated, optionally substituted C₁₋₄ hydrocarbon group,
X and Y are independently a hydroxyl group, an acetoxy group, a trifluoroacetoxy group, a tosyloxy group, a methanesulfonyloxy group, a trifluoromethanesulfonyloxy group, or a linear saturated halogenated C₁₋₁₀ hydrocarbon group;
in formula II, R₁ to R₅ are independently a hydrogen atom, halogen, a nitro group, a carboxyl group, or a linear or branched, saturated or unsaturated, optionally substituted C₁₋₄ hydrocarbon group; and
in formula III, R₁ to R₄ are independently a hydrogen atom, halogen, a nitro group, or a linear or branched, saturated or unsaturated, optionally substituted C₁₋₄ hydrocarbon group,
Z is halogen, a hydroxyl group, an acetoxy group, a trifluoroacetoxy group, or a linear or branched C₁₋₆ alkylperoxy group,
W₁ and W₂ are independently a methyl group, or W₁ and W₂ together form oxo (=O).

4. The oxidant according to claim 1, wherein the trivalent iodobenzene compound is selected from the group consisting of iodosobenzene, 2,4,6-trimethyl(diacetoxyiodo)benzene, [bis(trifluoroacetoxy)iodo]pentafluorobenzene, [hydroxy(tosyloxy)iodo]benzene, 2-iodosobenzoic acid, 1-fluoro-3,3-dimethyl-1,2-benziodoxol, and [hydroxy(methanesulfonyloxy)iodo]benzene.

5. The oxidant according to any one of claims 1-4, wherein the electron mediator is selected from the group consisting of a metal complex, a quinone compound, a phenazine compound, a viologen compound, a phenothiazine compound, and a phenol compound.

6. The oxidant according to claim 5, wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol.

7. A reagent for measurement of an analyte, containing an electron mediator, and the oxidant according to any one of claims 1-4.

8. The reagent for measurement of an analyte according to claim 7, further containing an enzyme that catalyzes oxidation reaction.

9. The reagent for measurement of an analyte according to claim 7 or 8, wherein the electron mediator is selected from the group consisting of a metal complex, a quinone compound, a phenazine compound, a viologen compound, a phenothiazine compound, and a phenol compound.

10. The reagent for measurement of an analyte according to claim 9, wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol.

11. A sensor comprising a working electrode, a counter electrode and a reagent layer, wherein the reagent layer contains an electron mediator and the oxidant according to any one of claims 1-4.

12. The sensor according to claim 11, wherein the reagent layer further contains an enzyme that catalyzes oxidation reaction.

13. The sensor according to claim 11 or 12, wherein the electron mediator is selected from the group consisting of a metal complex, a quinone compound, a phenazine compound, a viologen compound, a phenothiazine compound, and a phenol compound.

14. The sensor according to claim 13, wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol.

15. A method of electrochemically measuring an analyte, the method comprising:
(A) bringing a sample containing the analyte into contact with a reagent containing an electron mediator and the oxidant according to any one of claims 1-4, thereby the analyte is oxidized while the electron mediator is reduced, and
(B) using an electrode system comprising at least a working electrode and a counter electrode to obtain an electrochemical signal from the reduced electron mediator.

16. The method according to claim 15, wherein in step (A), the analyte is oxidized by the electron mediator while the electron mediator is reduced.

17. The method according to claim 15, wherein in step (A), the reagent further contains an enzyme that catalyzes oxidation reaction, and the analyte is oxidized by the enzyme while the electron mediator is reduced.

18. The method according to any one of claims 15-17, wherein the electron mediator is selected from the group consisting of a metal complex, a quinone compound, a phenazine compound, a viologen compound, a phenothiazine compound, and a phenol compound.

19. The method according to claim 18, wherein the electron mediator is at least one compound selected from the group consisting of potassium ferricyanide, hexaammineruthenium, ferrocene, poly(1-vinylimidazole)-bis-(bipyridine)chloroosmium, hydroquinone, 2-methyl-1,4-benzoquinone, 1,2-naphthoquinone-4-sulfonic acid or a salt thereof, 9,10-phenanthrenequinone-2-sulfonic acid or a salt thereof, 9,10-phenanthrenequinone-2,7-disulfonic acid or a salt thereof, 1,10-phenanthroline-5,6-dione, anthraquinone-2-sulfonic acid or a salt thereof, 1-methoxy-5-methylphenazinium methyl sulfate, hydroxyphenazine, methyl viologen, benzyl viologen, methylene blue, methylene green, 2-aminophenol, 2-amino-4-methylphenol, and 2,4-diaminophenol.
